# EUROPEAN PATENT SPECIFICATION

(11) **EP 1 920 796 B1**
(45) Date of publication and mention of the grant of the patent: **29.08.2012**
(21) Application number: 07119573.9
(22) Date of filing: 30.10.2007
(51) Int. Cl.: A61M 25/06

(54) **Safety cannula needle with needle protection element activated automaticly on detaching the needle from the catheter**
Sicherheitskanüle mit automatisch aktiviertem Nadelschutzelement bei der Nadelabnahme vom Katheter
Aiguille de canule de sécurité dotée d'un élément de protection d'aiguille automatiquement activé lors de la séparation de l'aiguille du cathéter

(30) Priority: 07.11.2006 IT MI20062127
(43) Date of publication of application: 14.05.2008
(73) Proprietor: ARTSANA S.p.A., 22070 Grandate (Como) (IT)
(72) Inventor: De Zolt, Dario, 22070, Appiano Gentile (Como) (IT)
(74) Representative: Ripamonti, Enrico

(56) References cited:
- WO-A-92/08502
- DE-U1- 20 218 551
- GB-A- 2 292 525
- US-A- 5 300 045

## Description

The present invention relates to a cannula needle or catheter needle in accordance with the introduction to the main claim.

A cannula needle of the aforedescribed type comprises a first and a second portion which are mutually separable. The first portion comprises a catheter (usually of biocompatible plastic) to be introduced into a patient's vein, for example of the arm, and secured to a member to be fixed (in known manner) to the patient, for example to the patient's arm, in proximity to that vein. The second portion comprises a metal cannula used to cut the epidermis and the vein, to enable the catheter tube to be inserted into the vein. The cannula is associated with its own support element.

When the catheter and cannula have been introduced into the vein, the cannula is extracted from the catheter and from the vein, and the second portion of the cannula needle is separated from the first. To this latter, or rather to the member carrying the catheter, there can then be connected any element, for example a tube, for transferring a medical product from a reservoir (for example a bag containing for example blood or nutrient fluid) into the vein.

The cannula needle can be of one or two way type, depending on whether the first portion must be able to be connected to one or two of said elements for transferring a medical product into the vein.

During the aforesaid separation of the needle of the second portion from the casing of the first portion it can happen that the medical operator or nurse assisting the patient may be accidentally pricked with the needle extracted from the catheter, with the possibility of contracting serious and dangerous infections.

The Applicant is aware of cannula catheter needles provided with hollow protection elements for containing the point of the needle after it has been separated from the catheter, to prevent the operator being pricked by the needle. Such elements are described for example in GB2292525 (which forms the state of the art to which the introduction to the main claim refers), US5300045 and

EP554841. Said elements are of more or less complicated form and are generally hollow and coupled to the cannula needle first portion when the second portion is associated with it: in other words, these elements are disposed between the two portions and are traversed by the cannula. When this is separated from the catheter the corresponding protection element is also separated from the first portion and lies on the point of the cannula, to cover it and protect the operator from contact therewith. For this purpose a movable element is positioned in the front of said point when positioned in the cavity of the protection element to close this cavity and prevent an operator from contacting the point.

Said protection elements can comprise elastic means which position them on the cannula, or are simply associated with the support for the needle such that they reach the point of this latter when it is separated from the catheter.

However, the known solutions do not always offer high reliability in use or are complicated and of considerable cost.

Moreover, the presence of a protection device with a movable element which closes onto the cavity of this element (or within it) in the front of the cannula point can interfere with its movement in separating from the catheter, making it more difficult (for example because of rubbing between this movable element and the catheter). Again, if this protection element is completely inside the catheter until final separation (as in US5300045), the operator may not be able to ascertain if the point of the cannula has been correctly protected or otherwise, on separation from the catheter.

An object of the present invention is to provide an improved catheter needle or cannula needle by which any risk of coming into contact with the cannula is safely and reliably eliminated, so ensuring the safety of those operators attending patients by adequately protecting them.

Another object is to provide a cannula needle of the stated type which is simple to produce and use, is of small size, and the use of which does not negatively affect the cannula needle operability and/or its separation from the catheter.

These and other objects which will be apparent to the expert of the art are attained by a cannula needle or catheter needle in accordance with the accompanying claims.

The present invention will be more apparent from the accompanying drawings which are provided by way of non-limiting example and in which:
Figure 1 is a perspective view of the overall catheter needle of the invention;
Figure 2 is a perspective view of a detail of the catheter needle of the invention;
Figure 3 is a detailed perspective view of a part of the invention;
Figure 4 is a partial longitudinal section through the detail of Figure 3;
Figure 5 is a partial perspective view of a stage during the use of the invention;
Figure 6 is a perspective longitudinal sectional view of the invention during the separation of the needle from the catheter;
Figure 7 is a longitudinal sectional side view of the invention during a further stage in its use;
Figure 8 is a perspective longitudinal sectional view of the invention during a stage in which its two parts are undergoing separation;
Figure 9 is a longitudinal section showing a further stage in the use of the invention;
Figure 10 is a perspective view of the invention with its two parts separated;
Figure 11 is an exploded perspective view of a specific embodiment of the invention;
Figure 12 is a perspective view of the part of Figure 11 assembled;
Figure 13 is a perspective view of a different embodiment of a part of the invention;
Figures 14 and 15 are perspective views from different angles of a further variant of a part of the invention;
Figure 16 is an exploded perspective view of a further embodiment of the invention;
Figure 17 is a partial section through a further variant of the invention;
Figure 18 is a section through a different internal configuration of the invention;
Figure 19 is a detail of Figure 18.

With reference to said figures, a cannula needle is indicated overall by 1 and comprises two portions 2 and 3, the first portion 2 being provided with a catheter 4 and the second portion comprising a usual cannula 5 to be inserted into the catheter 4 when the cannula has been introduced into patient's vein, and to be extracted from said catheter on termination of this introduction.

The first portion 2 comprises a known part 8 to be fixed to the patient's body in proximity to the vein and comprises a usual member 9 for securing it to a conduit (not shown) connected to a container or receiver (such as a bag) containing for example blood or another fluid. The second portion 3 comprises a usual support 10 for the cannula 5.

In known manner, the first portion 2 and second portion 3 are connected together on introducing the catheter 4 into the patient's vein, these portions being separable after this introduction to extract the cannula 5 from the catheter 4. The movement of the second portion 3 is facilitated by the presence of a gripping member 3A associated with this portion.

To prevent an operator from being pricked by the point 13 of the cannula 5, a protection element 15 is provided interposed between the portions 2 and 3. This protection element 15 receives the point 13 of the cannula 5 when this is extracted from the catheter 4 and withdrawn from the second portion 2, said point being completely covered by the element 15 to prevent its contact with said operator.

More specifically, the element 15 comprises a member 16 hollow at 17 and traversed by the cannula 5 when this is inserted into the catheter 4. The cavity 17 comprises a first portion 18 close to the second portion 3 of the cannula needle 1 and a second portion 19 provided in a part 16A of the member 16 to be inserted into the usual through hole 20 in the first portion of the cannula needle 1 traversed by the cannula 5. The second portion 19, open at 19K at the end of the part 16A of the member 16 facing the catheter, is substantially of funnel shape and tapers towards the first portion 18 of the cavity 17 from which it is separated by its reduced-diameter end 19A.

To enable the element 15 and the first portion 2 of the cannula needle 1 to be coupled together, the part 16A of the member 16 of this element 15 comprises an outer annular projection 22 which fits into a corresponding annular seat 23 provided in the wall 20A of the hole 20 in the first portion 2 of the cannula needle
1. In this manner, on introducing said part 16A of the member 16 into the hole 20, when the projection 22 fits into the seat 23 the element 15 and said first portion 2 are coupled together.

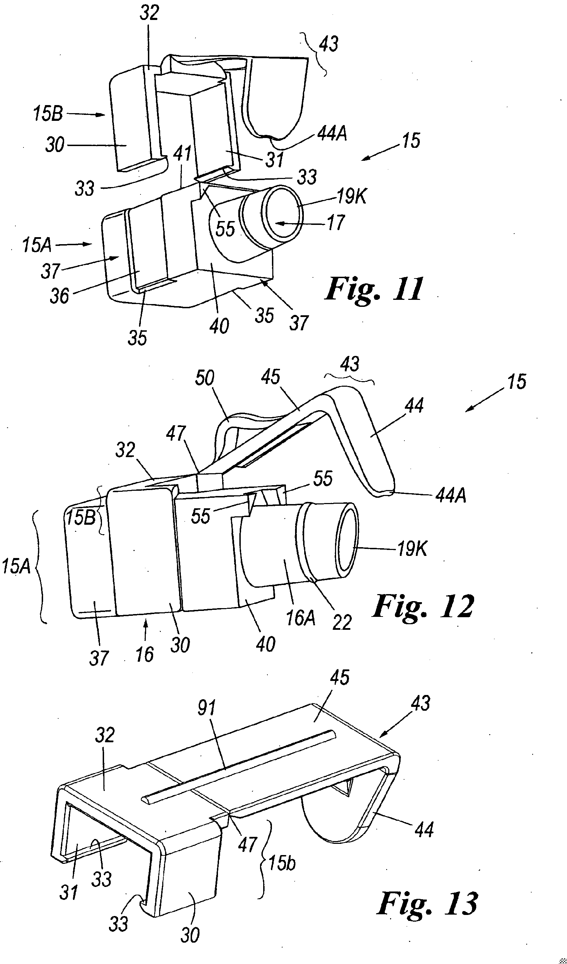

As shown in Figures 11 and 12, in a preferred embodiment of the invention, the element 15 comprises substantially two parts 15A, 15B coupled together by snap-engagement. Specifically, the part 15B is of substantially inverted U-shape and comprises two parallel arms 30, 31 joined together by a transverse part 32. Each of these arms presents an end 33 facing the opposing arm to cooperate by snap engagement with a corresponding seat 35 provided in the part 15A at the end of a corresponding groove 36 provide in a side 37 of said part. This groove 36 houses a corresponding arm 30, 31 of the part 15B. Preferably, to achieve this snap engagement, the distance between the arms 30, 31 is less than the distance between the sides 37 of the part 15A; in this manner, the arms 30, 31 diverge elastically on being mounted on the part 15A and approach each other when the ends 33 enter the seats 35 to hence lock the part 15B on the part 15A.

The part 15B can be of plastic, metal, or metal clad with or co-moulded with plastic or with any other material suitable for the purpose.

The part 15B can be mounted on the part 15A by mechanical coupling different from the aforesaid snap engagement.

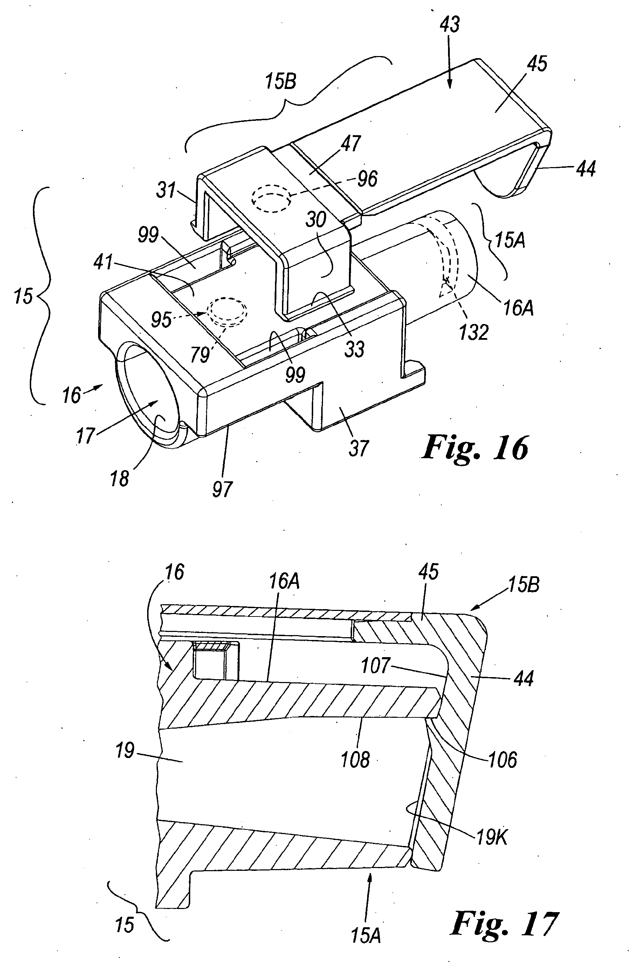

For example, as shown in Figure 16, the ends 33 of the two arms 30, 31 of the part 15B can face outwards. The arms 30, 31 of the portion 15B become inserted into two slots 99 opening, for example, into a side 41 perpendicular to the sides 37 of the part 15A. The ends 33 engage the part 15A such as to secure the part 15B to it. Alternatively, the slots 99 could be present in the sides 37.

The part 15B can present one or more holes 96 in its rear (with reference to the position of the element 15 relative to the catheter). Each hole couples to a corresponding pin 95 rising from the side 41 and having any shape. Each pin 95 can present, at its free end, an undercut 79 to more securely fix the part 15B to the part 15A by its insertion into the hole 96.

A further method for fixing the part 15B to the part 15A is by gluing or welding the two components together.

The part 15A, presenting the cavity 17, is of substantially parallelepiped shape and presents, projecting from a face 40 thereof, to which the sides are perpendicular, the part 16A of the member 16.

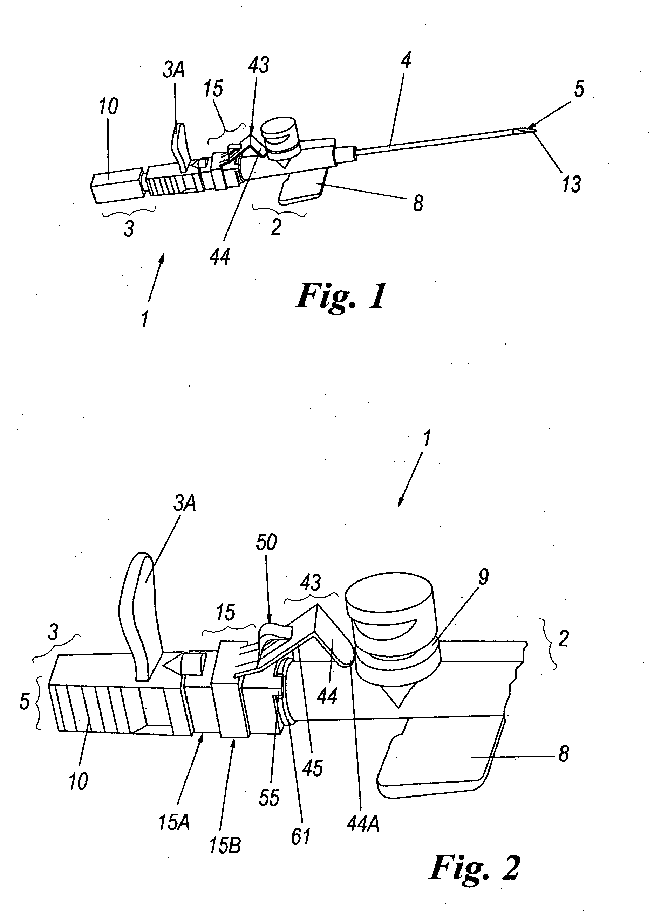

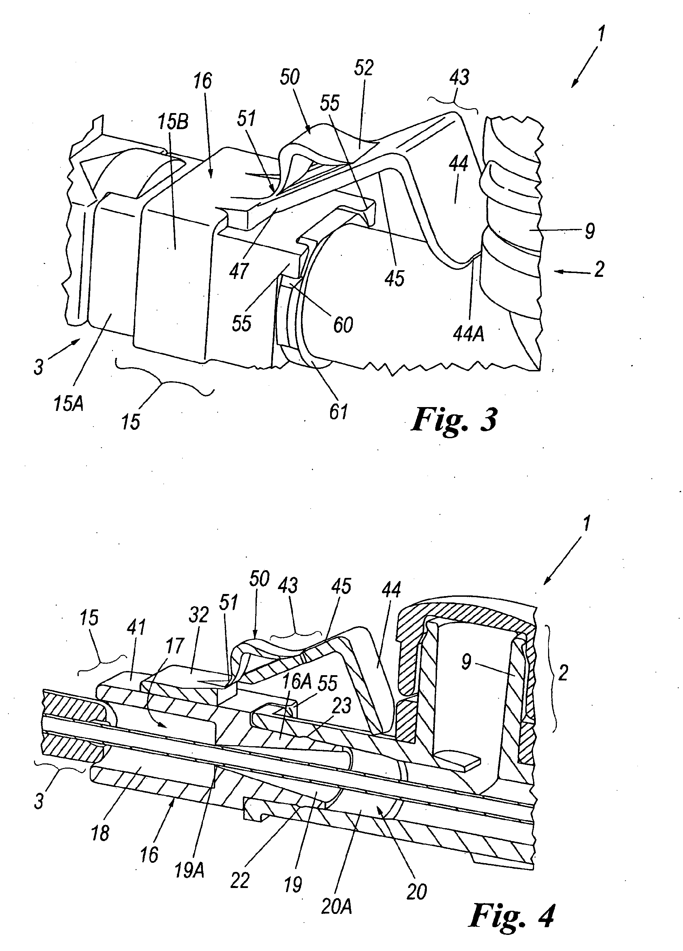

The transverse part 32 of the part 15B is positioned on an upper side 41 perpendicular to the sides 37 and to the face 40. From this transverse part there projects a substantially L-shaped piece 43 consequently presenting angled portions 44 and 45. The portion 45 is rigid with the part 32 via a hinge 47 formed by weakening material integral with the part 32 and the piece 43. Finally, in the embodiment of Figures 1-12, above this piece 43 there is a deformed elastic element 50 having a first end 51 rigid with the part 32 and a second end 52 acting on the portion 45 of the piece 43 and urging this latter towards the part 16A of the element 15.

On coupling the element 15 and the first portion 2 of the cannula needle 1 together, the portion 44 is urged by the elastic element 50 to bear on said first portion 2 and is maintained forcibly bearing against this latter. To improve this bearing, the free end 44A of the portion 44 is rounded or otherwise shaped to obtain a form fit with said portion 2.

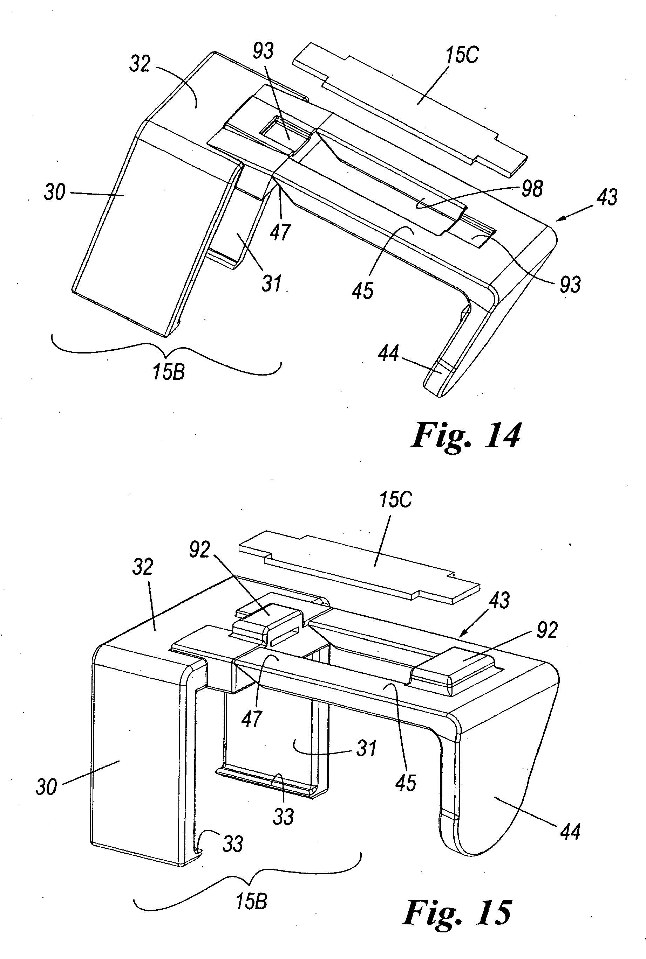

The elastic element 50 can be replaced by a metal component 15C having the same functions as the elastic element. This metal component 15C can have a shape suitable for securing to the piece 43 of the part 15B, or be simply a wire 91 as shown in Figure 13. If the metal component 15C has a substantially rectangular shape, as shown in Figures 14 and 15, it can be secured to the piece 43 of the part 15B by insertion into two opposing seats 93 present at the end of a recess (or of a through slot) 98 present within the portion 45. Alternatively, the component 15C can be inserted into two opposing pockets 92 present as projections on the parts 32 and 45 of the part 15B.

The component 15C can also be co-moulded with the part 15B.

If the piece 43 is made without any elastic element, as in Figure 16, and the part 15B is mainly of L-shape (although the part 32 and the arms 30, 31 are provided), the function of this elastic element is ensured by the mechanical and elastic properties of the constituent material of the part 15B and by the presence of the hinge 47. If the part 15B is made of metal (or metal clad with plastic), the function of the elastic element can be obtained by a particular configuration of the part 15B, for example bent about itself at the opposite end to the portion 44 and rigid with the hinge 47 (not shown). In this manner, its particular form offers preloading to the part 15B, to prevent its yielding with time, with the risk of the part 44 not coupling to the end 16A of the component 15A, so non longer ensuring complete safety to the operator.

The part 15B can also be made without any elastic element to automatically cause the portion 44 to rest on the end 19K of the element 15A. In that case, after extracting the needle, the operator must activate the safety system with a finger, to bring the portion 44 into contact with the end 19K.

The part 15A of the element 15 also comprises anti-twist means for its coupling to the portion 2 of the cannula needle 1. These anti-twist means are projections 55 extending from the face 40 of the portion 15A and arranged to cooperate with recesses 60 provided in a flange 61 present in known manner at the end of said portion 2.

In proximity to its point 13 at a predefined distance therefrom, the cannula 5 comprises a deformation 65 formed on the outside of the cannula to increase its transverse dimension. The distance of this transverse deformation from the point 13 is less than the length of the second portion 19 of the cavity 17 of the element 15 along the longitudinal axis W of this latter. On withdrawing the cannula from the catheter 4, this deformation cooperates by interference with the tapered end 19A of the portion 19, said cooperation preventing withdrawal of the needle from said element 15. This tapered end 19A is preferably flared at 70 on that side facing the first portion 18 of the cavity 17, to hence enable the cannula to pass towards the catheter 4 when the catheter needle 1 is being prepared for use, said inclination being such as to reduce the opening of the tapered end 19A in passing from the portion 18 to the portion 19 of the cavity 17. In this manner, the cannula can be introduced into the interior of the element 15 and can emerge therefrom by being moved towards the catheter 4, whereas it cannot pass through the cavity 17 in the reverse direction to completely withdraw from the element 15, as the deformation 65 of the cannula 5 makes contact with the tapered end 19A of the portion 19 of this cavity and engages it. It should also be noted that to insert the cannula 5 into the catheter 4 without problems while allowing said engagement between the deformation 65 and said end 19A, the opening or diameter of this latter is a few hundredths of a millimetre greater than the diameter of the cannula 5; this latter diameter is greater than that of said end at said deformation 65 where this diameter (of the cannula) is greater than that of the opening in said end 19A.

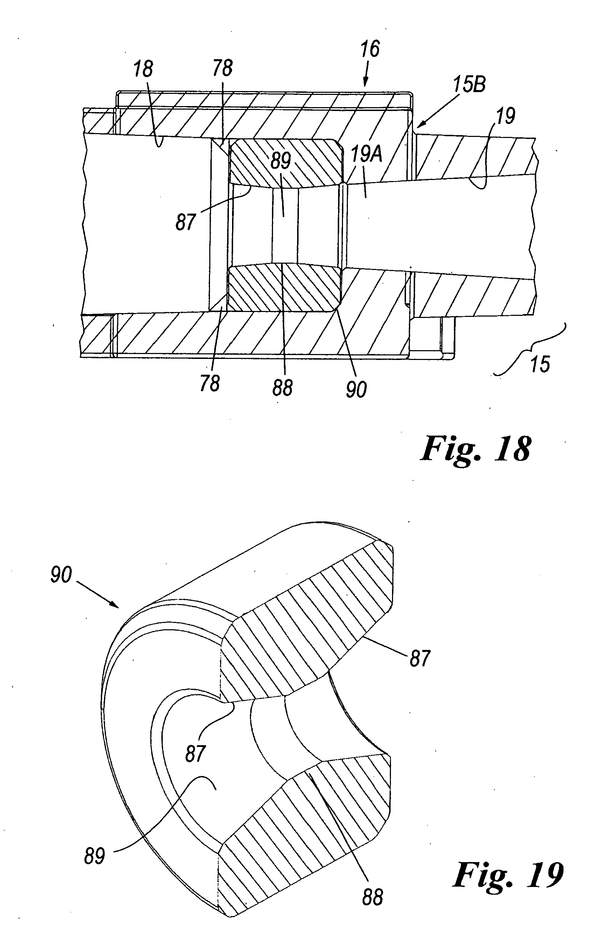

As an alternative to the tapered end 19A of the portion 19, a possible variant (shown in Figures 18 and 19) consists of inserting a ring or annular piece 90 into the cavity 18 of the part 15A of the protection element 15 within the portion 19 at a suitable distance from the aperture 19K. The annular piece 90 is hollow at 89 to enable the cannula 5 to slide within it. The central portion 88 of cavity 89 of annular piece 90 has a cross-section which is tenth microns greater than the nominal diameter of cannula 5, but less than the cross-section of the deformation 65 of the cannula 5. On withdrawing the cannula from the catheter 4, the deformation 65 cooperates by interference with the central portion 88 of the annular piece 90, said cooperation in no event enabling the needle to be withdrawn from said element 15.

The cavity of the annular piece presents a pair of flared portions 87 facing and tapering towards each other. This flared portion has the same function as the part 70, described in relation to the preceding figures.

The cavity of the annular piece 90 is symmetrical to enable said piece to be inserted into the cavity of the part 15A of the protection element 15 in both directions.

The piece 90 can be fixed inside the cavity 18 of the part 15A of the element 15 by interference, by making its diameter greater than the inner diameter of the cavity 18. Alternatively, an undercut 78 (in the form of an annular projection, for example see Figure 18) can be present inside the cavity 18 to maintain said piece 90 inside the cavity. Otherwise, the piece 90 can be glued to the interior of the element 15.

The annular piece can be made of plastic, metal, ceramic material or any other material of adequate hardness and rigidity.

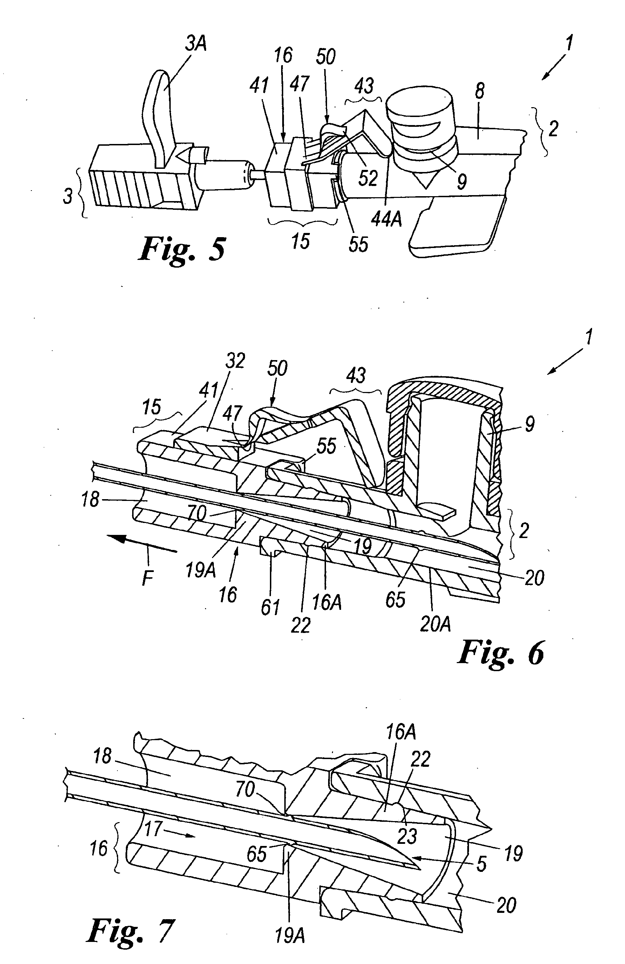

It will now be assumed that the cannula needle of the invention is to be used. After introducing the catheter 4 into the vein, the cannula 5 is extracted from it. In a first stage, visible in Figure 6, the needle is made to slide in the direction of the arrow F of that figure within the part 2 of the catheter needle 1 and into the cavity 17 of the element 15. This sliding takes place freely until the deformation 65 of the cannula 5 cooperates with the tapered end 19A (Figure 7), this cooperation blocking the cannula at this latter. In this position the point 13 of the cannula 5 has been completely inserted into the portion 19 of said cavity.

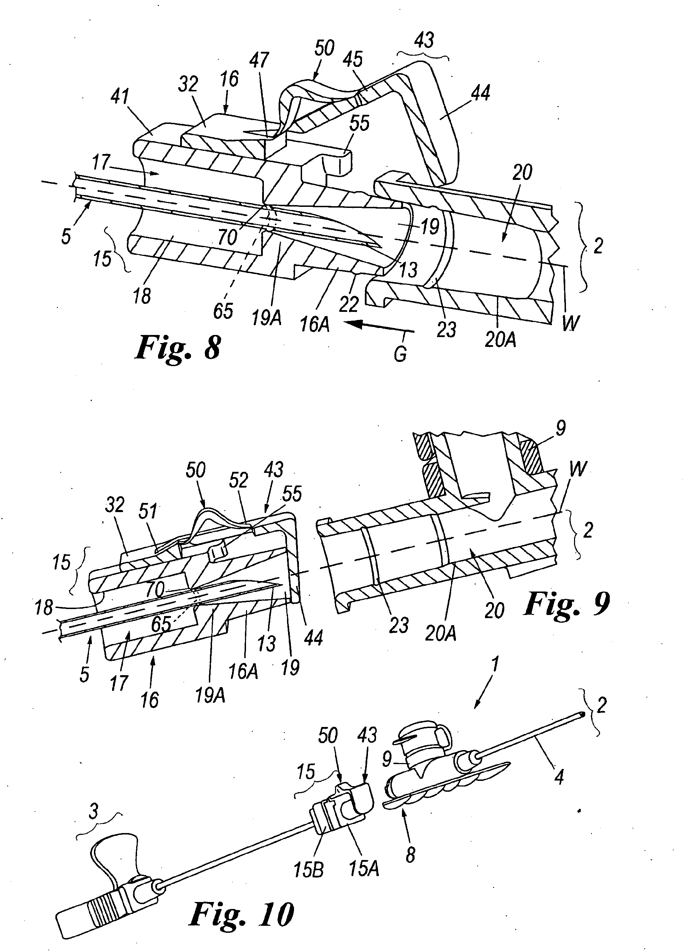

Continuing the needle traction, the force applied in the direction of the arrow G of Figure 8 (parallel to the arrow F of Figure 6) causes the annular projection 22 to detach from the corresponding seat 23, with consequent separation of the element 15 from the first portion 2 of the cannula needle 1. This element is hence detached from said portion.

On detachment, the elastic element 50 of the element 15 urges the portion 44 of the piece 43 above the aperture 19K of the portion 19 of the cavity 17 of the element 15, said portion 44 reclosing this aperture (and hence the part 16A of the member 16 of this element). As the point 13 of the cannula 5 is inserted into this portion and as the needle is stably coupled to the element 15, the closure of said aperture prevents an operator being able to touch the needle and be injured. This is achieved totally automatically and safely.

To make undesirable reopening of the cavity 17 more difficult, it is preferable to position the portions 44 and 45 of the piece 43 mutually inclined and non-perpendicular. In this manner, the portions 44 and 45 form an acute angle between themselves, with the portion 44, after closure of the cavity, lying tightly resting on the aperture 19K. Likewise, the aperture 19K can be formed not in a plane perpendicular to the axis W, but inclined to the axis W by an angle equal to the angle between the parts 44 and 45 of the piece 43. This aperture hence follows the inclination of the portion 44 for better closure.

To render the invention even more safe, mechanical couplings can be formed between the portion 44 and the part 16a of the member 16 of the element 15 to achieve snap closure of the aperture 19K, as shown in Figure 17.

This figure shows a safety mechanism consisting of a triangular protrusion 106 positioned on the inner surface 107 of the part 44 of the piece 43 of the part 15B. When the part 44 covers the aperture 19K of the piece 43, this protrusion 106 irreversibly engages the surface 108 of the portion 19 of the part 15A.

If the piece 43 is of steel, the triangular protrusion can be obtained by local deformation of the part 44 of said piece.

Safety can be further assured by a slot 132 (for example shown in Figure 16) provided in the portion 19 of the part 15A of the element 15. This slot is arranged to receive the part 44 of the piece 43 when positioned in front of the aperture 19K. The invention is hence of simple construction and use, and is of very low production cost. In particular, as the protection element 15 is in the form of two separate portions 15A, 15B, it is simple to construct and to assemble with a cannula needle. The element can be produced separately from the cannula needle and then be connected to it, this allowing considerable flexibility from the industrial viewpoint.

The two parts are easy to fit together using various industrial solutions, hence enabling the producer to choose the optimum solution.

Moreover, as the two parts are separate, they can be made of different materials, hence using materials suitable for the different functions which these parts have to perform.

Various preferred embodiments of the invention have been described; others are however possible in the light of the aforegoing: for example, the element 15 can be torsionally locked onto the portion 2 of the cannula needle in a manner different from that described (for example, coupling by inserting pins into seats provided in the two components of the cannula needle) or the element 15 can be coupled to the portion 2 by external snap-engagement without providing the projection 22 cooperating with the seat 23. Alternatively, the protection element 15 could be coupled to the front portion 2 of the cannula needle 1, or the projection 22 cooperating with the seat 23 could be replaced by other mechanical couplings, already known to those operating in this sector.

According to other variants of the invention the part 15B of the protection element 15 is defined by just the L-shaped piece, which is associated at its free end with the part 15A of said element.

This free end bent on itself to thicken it, is inserted into a seat provided in a raised portion formed on the side 41 of that part. As an alternative, said end can present a hole (as the hole 96 of Fig. 16) and is arranged to cooperate with a pin (as pin 95 of Fig. 16) to secure it to the side 41 of the part 15A of the element 15.

These variants are also to be considered as falling within the scope of the present document as defined by the accompanying claims.

## Claims

1. A cannula needle or catheter needle (1) comprising a first portion (2) provided with a catheter (4) to be introduced into a vein and a second portion (3) provided with a cannula (5) which is inserted into the catheter (4) on introducing this latter into the vein, but is separable to be withdrawn from it after this introduction has taken place, the cannula (5) provided with a point (13) being associated with its own support (10) and being arranged to cooperate with a protection element (15) presenting a member (16) hollow (at 17) and open (at 19K) towards the catheter into which said cannula (5) is through-inserted, the cannula being locked in said element (15) when separated from the catheter (4), the protection element (15) being coupled to the first portion (2) and being interposed between this latter and said second portion (3), said protection element (15) being separable from the first portion (2) during the withdrawal of the cannula (5) from the catheter (4), said cannula (5) comprising coupling means (65) for securing it to said element (15) when its point (13), withdrawn from the catheter (4), is totally inserted into said cavity (17), this cooperation, together with the movement of the cannula (5), causing the protection element (15) to become detached from said first portion (2) of the cannula needle (1), the protection element (15) comprising a closure part (43) for closing its cavity (17) in which the cannula (5) is disposed on detaching said element (15) from the first portion (2) of the cannula needle (1), said protection element (15) being mechanically coupled to a part of the first portion (2) of said cannula needle (1), said closure part (43) being L-shaped and presenting angled portions (44, 45), a first (44) of these latter being arranged to position itself in front of the cavity (17) of the protection element (15) at the moment at which this latter separates from the first portion (2) of the cannula needle (1), **characterised in that** the closure part (43) is L-SHAPED and forms at least part of a first portion (15B) of the protection element (15) which becomes connected to a second portion (15A) of the protection element (15) in which the internal cavity (17) of said element (15) is present, a part (32) of the first portion (15B) being fixed to the second portion (15A).

2. A cannula needle as claimed in claim 1, **characterised in that** said L-shaped part (43) defines said first portion (15B) of the protection element and is directly secured to the second portion (15A) of this latter.

3. A cannula needle as claimed in claim 2, **characterised in that** said L-shaped part (43) is an I-shaped strip and is secured at a free end to a retention element associated with the second portion (15A) of said protection element.

4. A cannula needle as claimed in claim 1, **characterised in that** the first portion (15B) is substantially of inverted U-shape and comprises two arms (30, 31) arranged to connect to the second portion (15A) by mechanical coupling, said arms being connected together by the transverse part (32) from which the L-shaped part (43) projects.

5. A cannula needle as claimed in claim 4, **characterised in that** said arms (30, 31) present ends (33) arranged to snap-engage respective seats (35, 99) provided inn the second portion (15A) of the protection element.

6. A cannula needle as claimed in claim 4, **characterised in that** the first portion (44) of the L-shaped piece (43) is elastically loaded and presents an end (44A) which engages as a form fit with the exterior of the first portion (2) of the cannula needle (1) on which this end presses when the protection element is rigid with said first portion (2) of the cannula needle (1).

7. A cannula needle as claimed in claim 1, **characterised by** comprising means (106) for mechanical connection between the first portion (44) of the part (43) and that part (16A) of the member (16) of the protection element (15) provided with the aperture (19K), said means (106) being associated with an internal surface (107) of said portion (44) and cooperating in closure with the internal surface (108) such as to maintain said portion (44) closed onto said aperture.

8. A cannula needle as claimed in claim 1, **characterised in that** the portions (44, 45) of the part (43) form a contained acute angle.

9. A cannula needle as claimed in claim 1, **characterised in that** the protection element (15) is mechanically coupled to an internal part (20) of said first portion (2).

10. A cannula needle as claimed in claim 9, **characterised in that** the member (16) of the protection element (15) presents a part (16A) inserted into the usual through hole (20) of said first portion (2) of the cannula needle, said part (16A) comprising coupling means (22) and restraining counter-means (23) provided in said hole (20).

11. A cannula needle as claimed in claim 10, **characterised in that** the coupling means are an annular projection (22) provided on the outside of the part (16A) of the member (16) of the protection element (15), the counter-means being a seat (23) provided within the wall (20A) of said through hole (20).

12. A cannula needle as claimed in claim 10, **characterised in that** the cavity (17) of said member (16) comprises two consecutive portions (18, 19), the first portion (18) facing the second portion (3) of the cannula needle, the second portion (19) being tapered towards the first and terminating with a reduced diameter end (19A) separating the second portion (19) from the first (18), the second portion having an aperture (19K) at that end (16A) of the member (16) inserted into the through hole (20) of the second portion (2) of the cannula needle (1).

13. A cannula needle as claimed in claim 12, **characterised in that** the reduced diameter end (19A) is defined by an annular piece (90) internally hollow (at 89) and presenting flared portions (87) which taper towards each other.

14. A cannula needle as claimed in claim 13, **characterised in that** the annular piece (90) is fixed within the protection element.

15. A cannula needle as claimed in claim 1, **characterised in that** the closure piece (43) of the first portion (15B) of the protection element (15) is elastically urged to bring the first (44) of its portions in front of the cavity (17) of the protection element (15), when in this position it being positioned in front of the aperture (19K) of said cavity (17), facing the first portion (2) of the cannula needle (1).

16. A cannula needle as claimed in claim 15, **characterised in that** the protection element (15) comprises an elastic element (50, 15C) rigid with the first portion (15B) of the protection element (15) and having a second end (52) acting on the part (45) of the L-shaped piece (43) to urge the first portion (44) of this latter in front of the cavity (17).

17. A cannula needle as claimed in claim 1, **characterised in that** the closure piece (43) of the first portion (15B) of the protection element (15) is elastically urged to bring the first (44) of its portions (44, 45) into a slot (132) provided in the second portion (15A) and opening into the cavity (17) of this latter.

18. A cannula needle as claimed in claim 15, **characterised in that** the elastic element (50) is integral with the first portion (15B) of the protection element.

19. A cannula needle as claimed in claim 15, **characterised in that** the elastic element is a component (15C) independently coupled to and rigid with the first portion (15B) of said element so as to act on the L-shaped piece (43) of this latter.

20. A cannula needle as claimed in claim 18, **characterised in that** said elastic element is at least partially inserted in the seat and/or housings (93, 98) of said first portion (15B).

21. A cannula needle as claimed in claim 1, **characterised in that** the aperture (19K) **in that** part (16A) of the member (16) facing the catheter lies in a plane cutting the axis (W), this aperture (19K) being inclined to the axis (W) by an angle equal to the angle between the parts (44, 45) of the piece (43).

22. A cannula needle as claimed in claim 1, **characterised in that** anti-twist means (55) are provided on the protection element (15) to cooperate with counter-means (60) of the first portion (2) of the cannula needle to prevent relative torsional movements between this latter and said element.

23. A cannula needle as claimed in claim 22, **characterised in that** the anti-twist means are at least one arm (55) projecting from the protection element and arranged to cooperate with a recess (60) provided in an end flange (61) of said first portion (2) of the cannula needle (1).

24. A cannula needle as claimed in claim 23, **characterised in that** the anti-twist means comprising the arm (55) present a snap coupling to couple the protection element (15) to the front portion (2) of said cannula needle (1).

25. A cannula needle as claimed in claim 22, **characterised in that** the anti-twist means are pins which become inserted into seats provided in the first and in the second portion (2, 3) of the cannula needle.

## Patentansprüche

1. Eine Kanülen- oder Katheternadel (1), die einen ersten Abschnitt (2) umfasst, welcher mit einem Katheter (4) zur Einführung in eine Vene ausgerüstet ist, und einen zweiten Abschnitt (3), der mit einer Kanüle (5) versehen ist, die in den Katheter (4) bei Einführung dieses letzteren in die Vene eingesetzt wird, aber trennbar daraus zurückgezogen werden kann, nachdem diese Einführung stattgefunden hat, wobei die Kanüle (5) mit einer Spitze 13) ausgestattet ist, die jeweils mit einer eigenen Halterung (10) verbunden ist, und dabei so angeordnet ist, dass sie mit einem Schutzelement (15) zusammenarbeitet, welches ein Glied (16) aufweist, das hohl (bei 17) und offen (bei 19K) in Richtung des Katheters ist, in den die genannte Kanüle (5) eingesetzt wird, wobei die Kanüle in dem genannten Element (15) verriegelt wird, wenn sie von dem genannten Katheter (4) getrennt wird, und das Schutzelement (15) mit dem ersten Abschnitt (2) verbunden und zwischen diesen letzteren und den genannten zweiten Abschnitt geschoben wird, wobei das genannte Schutzelement (15) von dem ersten Abschnitt (2) während der Rückzugsphase der Kanüle (5) vom Katheter (4) getrennt werden kann, und die genannte Kanüle (5) jeweils Kupplungselemente (65) umfasst, um sie mit dem genannten Element (15) zu verbinden, wenn ihre Spitze (13), die sich vom Katheter (4) zurückgezogen hat, ganz in den genannten Hohlraum (17) eingeschoben ist, wobei diese Zusammenarbeit, zusammen mit der Bewegung der Kanüle (5) das Schutzelement (15) in die Lage versetzt, sich von dem genannten ersten Abschnitt (2) der Kanülennadel (1) zu trennen, und das Schutzelement (15) ein Verschlussteil (43) zum Verschließen seines Hohlraums (17) umfasst, in dem die Kanüle (5) angeordnet ist, wenn das genannte Element (15) vom ersten Abschnitt (2) der Kanülennadel (1) getrennt wird, wobei das genannte Schutzelement mit einem Teil des ersten Abschnitts (2) der genannten Kanülennadel mechanisch verbunden ist, und das genannte Verschlussteil (43) L-förmig ist sowie winklige Abschnitte (44, 45) aufweist, und ein erster (44) dieser letzteren so angeordnet ist, dass er gegenüber des Hohlraums (17) des Schutzelements (15) zu liegen kommt, und zwar in dem Augenblick, in dem dieses letztere sich vom ersten Abschnitt (2) der Kanülennadel (1) trennt, **dadurch gekennzeichnet, dass** das Verschlussteil (43) L-förmig ist und mindestens einen Teil des ersten Abschnitt (15B) des Schutzelements (15) bildet, welches mit einem zweiten Abschnitt (15A) des Schutzelements (15) verbunden wird, in dem der innere Hohlraum (17) des genannten Elements (15) vorhanden ist, wobei jeweils ein Teil (32) des ersten Abschnitts (15B) am zweiten Abschnitt (15A) befestigt ist.

2. Eine Kanülennadel gemäß Anspruch 1, **dadurch gekennzeichnet, dass** das genannte L-förmige Teil (43) den genannten ersten Abschnitt (15B) des Schutzelements bildet und direkt an dem zweiten Abschnitt (15A) dieses letzteren befestigt ist.

3. Eine Kanülennadel gemäß Anspruch 2, **dadurch gekennzeichnet, dass** das genannte L-förmige Teil (43) ein 1-förmiger Streifen ist und an einem freien Ende an einem Rückhalteelement befestigt ist, das mit dem zweiten Abschnitt (15A) des genannten Schutzelements verbunden ist.

4. Eine Kanülennadel gemäß Anspruch 1, **dadurch gekennzeichnet, dass** der erste Abschnitt (15B) im wesentlichen eine umgekehrte U-Form aufweist und zwei Arme umfasst (30, 31), welche so angeordnet sind, dass sie jeweils mit dem zweiten Abschnitt (15A) durch eine mechanische Kupplung verbunden sind, wobei die genannten Arme jeweils miteinander durch das Querteil (32) verbunden sind, von dem das L-förmige Teil hervorragt.

5. Eine Kanülennadel gemäß Anspruch 4, **dadurch gekennzeichnet, dass** die genannten Arme (30, 31) Enden (33) aufweisen, die so angeordnet sind, dass sie jeweils mit entsprechenden Sitzen (35, 99) einrasten, die am zweiten Abschnitt (15A) des Schutzelements vorgesehen sind.

6. Eine Kanülennadel gemäß Anspruch 4, **dadurch gekennzeichnet, dass** der erste Abschnitt (44) des L-förmigen Teils (43) elastisch belastet ist und ein Ende (44A) aufweist, das mit dem Äußeren des ersten Abschnitts (2) der Kanülennadel (1) formschlüssig einrastet, auf die dieses Ende drückt, wenn das Schutzelement mit dem genannten ersten Abschnitt (2) der Kanülennadel (1) starr verbunden ist.

7. Eine Kanülennadel gemäß Anspruch 1, **dadurch gekennzeichnet, dass** sie Elemente (106) für die mechanische Verbindung zwischen dem ersten Abschnitt (44) des Teils (43) und dem Teil (16A) des Glieds (16) des Schutzelements (15) umfasst, das mit der Öffnung (19K) versehen ist, wobei die genannten Elemente (106) mit einer inneren Oberfläche (107) des genannten Abschnitts (44) verbunden sind und bei Schließung mit der inneren Oberfläche (108) zusammenarbeiten, um den genannten Abschnitt (44) geschlossen auf der genannten Öffnung zu halten.

8. Eine Kanülennadel gemäß Anspruch 1, **dadurch gekennzeichnet, dass** die Abschnitte (44, 45) des Teils (43) einen eingeschlossenen spitzen Winkel bilden.

9. Eine Kanülennadel gemäß Anspruch 1, **dadurch gekennzeichnet, dass** das Schutzelement (15) mechanisch mit einem Innenteil (20) des genannten ersten Abschnitts (2) verbunden ist.

10. Eine Kanülennadel gemäß Anspruch 9, **dadurch gekennzeichnet, dass** das Glied (16) des Schutzelements (15) ein Teil (16A) aufweist, das in das übliche durchgehende Loch (20) des genannten ersten Abschnitts (2) der Kanülennadel eingesetzt ist, wobei das genannte Teil (16A) Kupplungselemente (22) und Rückhalte-Gegenelemente (23) umfasst, die in diesem Loch (20) vorgesehen sind.

11. Eine Kanülennadel gemäß Anspruch 10, **dadurch gekennzeichnet, dass** die Kupplungselemente ein ringförmiger Vorsprung (22) sind, der an der Außenseite des Teils (16A) des Glieds (16) des Schutzelements (15) vorgesehen ist, wobei die Gegenelemente ein Sitz (23) sind, der innerhalb der Wand (20A) des genannten durchgehenden Lochs (20) vorgesehen ist.

12. Eine Kanülennadel gemäß Anspruch 10, **dadurch gekennzeichnet, dass** der Hohlraum (17) des genannten Glieds (16) zwei aufeinanderfolgende Abschnitte (18, 19) umfasst, wobei der erste Abschnitt (18) dem zweiten Abschnitt (3) der Kanülennadel gegenüberliegt, und der zweite Abschnitt (19) sich in Richtung des ersten verjüngt und mit einem Ende mit geringerem Durchmesser (19A) endet, wobei der zweite Abschnitt (19) vom ersten (18) getrennt wird und der zweite Abschnitt eine Öffnung (19K) am Ende (16A) des Glieds (16) aufweist, das in das durchgehende Loch (20) des zweiten Abschnitts (2) der Kanülennadel (1) eingesetzt wird.

13. Eine Kanülennadel gemäß Anspruch 12, **dadurch gekennzeichnet, dass** das Ende mit geringerem Durchmesser (19A) durch ein innen hohles (bei 89) ringförmiges Teil (90) gebildet wird und ausgehalste Abschnitte (87) aufweist, die sich jeweils gegeneinander verjüngen.

14. Eine Kanülennadel gemäß Anspruch 13, **dadurch gekennzeichnet, dass** das ringförmige Teil (90) im Schutzelement befestigt ist.

15. Eine Kanülennadel gemäß Anspruch 1, **dadurch gekennzeichnet, dass** das Verschlussteil (43) des ersten Abschnitts (15B) des Schutzelements (15) elastisch veranlasst wird, den ersten (44) seiner Abschnitte jeweils gegenüber dem Hohlraum (17) des Schutzelements anzuordnen, wenn es in dieser Stellung gegenüber der Öffnung (19K) des genannten Hohlraums zu liegen kommt und dabei dem ersten Abschnitt (2) der Kanülennadel (1) gegenüberliegt.

16. Eine Kanülennadel gemäß Anspruch 15, **dadurch gekennzeichnet, dass** das Schutzelement (15) ein elastisches Element (50, 15C) umfasst, das starr mit dem ersten Abschnitt (15B) des Schutzelements (15) verbunden ist und ein zweites Ende (52) aufweist, welches auf das Teil (45) des L-förmigen Teils (43) einwirkt, um den ersten Abschnitt (44) dieses letzteren zu veranlassen, sich gegenüber vom Hohlraum (17) anzuordnen.

17. Eine Kanülennadel gemäß Anspruch 1, **dadurch gekennzeichnet, dass** das Verschlussteil (43) des ersten Abschnitts (15B) des Schutzelements (15) elastisch veranlasst wird, jeweils den ersten (44) seiner Abschnitte (44, 45) in einem entsprechenden Schlitz (132) unterzubringen, der in dem zweiten Abschnitt (15A) vorgesehen ist und sich in den Hohlraum (17) dieses letzteren hinein öffnet.

18. Eine Kanülennadel gemäß Anspruch 15, **dadurch gekennzeichnet, dass** das elastische Element (50) einteilig mit dem ersten Abschnitt (15B) des Schutzelements ist.

19. Eine Kanülennadel gemäß Anspruch 15, **dadurch gekennzeichnet, dass** das elastische Element ein Bauteil (15C) ist, das unabhängig und starr mit dem ersten Abschnitt (15B) des genannten Elements verbunden ist, so dass es auf das L-förmige Tel (43) dieses letzteren einwirkt.

20. Eine Kanülennadel gemäß Anspruch 18, **dadurch gekennzeichnet, dass** das genannte elastische Element jeweils mindestens teilweise in den Sitz und/oder die Gehäuse (93, 98) des genannten ersten Abschnitts (15B) eingesetzt ist.

21. Eine Kanülennadel gemäß Anspruch 1, **dadurch gekennzeichnet, dass** die Öffnung (19K) in diesem Teil (16A) des Glieds (16), das dem Katheter gegenüberliegt, auf einer Ebene liegt, die die Achse (W) schneidet, wobei diese Öffnung (19K) jeweils im Verhältnis zur Achse (W) in einem Winkel geneigt ist, der dem Winkel zwischen den Teilen (44, 45) des Teils (43) entspricht.

22. Eine Kanülennadel gemäß Anspruch 1, **dadurch gekennzeichnet, dass** Verdrehungsschutzelemente (55) am Schutzelement (15) vorgesehen sind, um jeweils mit den Gegenelementen (60) des ersten Abschnitts (2) der Kanülennadel zusammenzuarbeiten und die entsprechenden Drehbewegungen zwischen dieser letzteren und dem genannten Element zu verhindern.

23. Eine Kanülennadel gemäß Anspruch 22, **dadurch gekennzeichnet, dass** die Verdrehungsschutzelemente aus mindestens einem Arm (55) bestehen, der jeweils von dem Vorsprungselement hervorragt und so angeordnet ist, dass er mit einer Aussparung (60) zusammenarbeitet, welche sich an einem Endflansch (61) des genannten ersten Abschnitts (2) der Kanülennadel (1) befindet.

24. Eine Kanülennadel gemäß Anspruch 23, **dadurch gekennzeichnet, dass** die Verdrehungsschutzelemente, die den Arm (55) umfassen, jeweils eine Schnappkupplung aufweisen, um das Schutzelement (15) mit dem vorderen Abschnitt (2) der genannten Kanülennadel (1) zu verbinden.

25. Eine Kanülennadel gemäß Anspruch 22, **dadurch gekennzeichnet, dass** die Verdrehungsschutzelemente Nadeln sind, die in entsprechende Sitze eingesetzt werden, welche jeweils am ersten und zweiten Abschnitt (2, 3) der Kanülennadel vorgesehen sind.

## Revendications

1. Aiguille en forme de canule ou aiguille en forme de cathéter (1) comprenant une première portion (2) pourvue d'un cathéter (4) devant être introduit dans une veine et une deuxième partie (3) pourvue d'une canule (5) qui est introduite dans le cathéter (4) lors de l'introduction de ce dernier dans la veine, mais pouvant être séparée pour être retirée de celui-ci après que cette introduction ait eu lieu, la canule (5) pourvue d'une pointe (13) étant associée à son propre support (10) et étant réalisée de sorte qu'elle coopère avec un élément de protection (15) présentant un membre (16) creux (en 17) et ouvert (en 19K) en direction du cathéter, dans lequel ladite canule (5) est introduite par passage, la canule étant verrouillée dans ledit élément (15) quand elle est séparée du cathéter (4), l'élément de protection (15) étant accouplé avec la première portion (2) et étant interposé entre cette première et ladite deuxième portion (3), ledit élément de protection (15) pouvant être séparé de la première portion (2) pendant le retrait de la canule (5) du cathéter (4), ladite canule (5) comprenant des moyens d'accouplement (65) pour la fixer audit élément (15) quand sa pointe (13), retirée du cathéter (4), est totalement introduite dans ladite cavité (17), cette coopération, avec le mouvement de la canule (5), entraînant le détachement de l'élément de protection (15) de ladite première portion (2) de l'aiguille en forme de canule (1), l'élément de protection (15) comprenant une partie de fermeture (43) pour fermer la cavité (17), dans laquelle la canule (5) est placée lors du détachement dudit élément (15) de la première portion (2) de l'aiguille en forme de canule (1), ledit élément de protection (15) étant mécaniquement accouplé avec une partie de la première portion (2) de ladite l'aiguille en forme de canule (1), ladite partie de fermeture (43) étant réalisée en forme de L et présentant des portions à angle (44, 45), une première portion (44) de ces dernières étant réalisée pour se placer devant la cavité (17) de l'élément de protection (15) au moment où ce dernier se sépare de la première portion (2) de l'aiguille en forme de canule (1), **caractérisée en ce que** la partie de fermeture (43) est réalisée en forme de L et qu'elle forme au moins une partie de la première portion (15B) de l'élément de protection (15) qui va se raccorder à la deuxième portion (15A) de l'élément de protection (15), où la cavité interne (17) dudit élément (15) est présente, une partie (32) de la première portion (15B) étant fixée à la deuxième portion (15A).

2. Aiguille en forme de canule selon la revendication 1, **caractérisée en ce que** ladite partie en forme de L (43) définit ladite première portion (15B) de l'élément de protection et qu'elle est directement fixée à la deuxième portion (15A) de ce dernier.

3. Aiguille en forme de canule selon la revendication 2, **caractérisée en ce que** ladite partie en forme de L (43) est une bande en forme de I et qu'elle est fixée à l'extrémité libre à un élément de rétention associé à la deuxième portion (15A) dudit élément de protection.

4. Aiguille en forme de canule selon la revendication 1, **caractérisée en ce que** ladite première portion (15B) est substantiellement réalisée en forme de U inversé et qu'elle comprend deux bras (30, 31) prévus pour raccorder la deuxième portion (15A) par accouplement mécanique, lesdits bras étant reliés entre eux par la partie transversale (32) à partir de laquelle la partie en forme de L (43) sort en saillie.

5. Aiguille en forme de canule selon la revendication 4, **caractérisée en ce que** lesdits bras (30, 31) présentent des extrémités (33) réalisées pour s'engager à coup sec dans des logements respectifs (35, 99), prévus dans la deuxième portion (15A) de l'élément de protection.

6. Aiguille en forme de canule selon la revendication 4, **caractérisée en ce que** la première portion (44) de la pièce en forme de L (43) est chargée de façon élastique et présente une extrémité (44A) qui s'engage comme une forme s'adaptant avec l'extérieur de la première portion (2) de l'aiguille en forme de canule (1), sur laquelle cette extrémité appuie quand l'élément de protection est rigide avec ladite première portion (2) de l'aiguille en forme de canule (1).

7. Aiguille en forme de canule selon la revendication 1, **caractérisée en ce qu'**elle comprend des moyens (106) de raccord mécanique entre la première portion (44) de la partie (43) et la partie (16A) du membre (16) de l'élément de protection (15), pourvu de l'ouverture (19K), lesdits moyens (106) étant associés à une surface interne (107) de ladite portion (44) et coopérant en fermeture avec la surface interne (108) de façon à maintenir ladite portion (44) fermée sur ladite ouverture.

8. Aiguille en forme de canule selon la revendication 1, **caractérisée en ce que** les portions (44, 45) de la partie (43) forment un angle aigu inclus.

9. Aiguille en forme de canule selon la revendication 1, **caractérisée en ce que** l'élément de protection (15) est mécaniquement accouplé à une partie interne (20) de ladite première portion (2).

10. Aiguille en forme de canule selon la revendication 9, **caractérisée en ce que** le membre (16) de l'élément de protection (15) présente une partie (16A) introduite dans le trou passant traditionnel (20) de ladite première portion (2) de l'aiguille en forme de canule, ladite partie (16A) comprenant des moyens d'accouplement (22) et des contre-moyens de restriction (23) prévus dans ledit trou (20).

11. Aiguille en forme de canule selon la revendication 10, **caractérisée en ce que** les moyens d'accouplement sont une saillie annulaire (22) prévue sur l'extérieur de la partie (16A) du membre (16) de l'élément de protection (15), les contre-moyens étant un logement (23) prévu à l'intérieur de la paroi (20A) de ledit trou passant (20).

12. Aiguille en forme de canule selon la revendication 10, **caractérisée en ce que** la cavité (17) dudit membre (16) comprend deux portions consécutives (18, 19), la première portion (18) faisant face à la deuxième portion (3) de l'aiguille en forme de canule, la deuxième portion (19) étant fuselée en direction de la première et terminant par une extrémité au diamètre réduit (19A) séparant la deuxième portion (19) de la première (18), la deuxième portion présentant une ouverture (19K) à cette extrémité (16A) du membre (16), introduite dans le trou passant (20) de la deuxième portion (2) de l'aiguille en forme de canule (1).

13. Aiguille en forme de canule selon la revendication 12, **caractérisée en ce que** l'extrémité au diamètre réduit (19A) est définie par une pièce annulaire (90) creuse à l'intérieur (en 89) et présentant des portions évasées (87) qui sont fuselées l'une en direction de l'autre.

14. Aiguille en forme de canule selon la revendication 13, **caractérisée en ce que** la pièce annulaire (90) est fixée à l'intérieur de l'élément de protection.

15. Aiguille en forme de canule selon la revendication 1, **caractérisée en ce que** la pièce de fermeture (43) de la première portion (15B) de l'élément de protection (15) est poussée de façon élastique pour porter la première (44) de ses portions devant la cavité (17) de l'élément de protection (15), quand dans cette position elle est placée devant l'ouverture (19K) de ladite cavité (17), faisant face à la première portion (2) de l'aiguille en forme de canule (1).

16. Aiguille en forme de canule selon la revendication 15, **caractérisée en ce que** l'élément de protection (15) comprend un élément élastique (50, 15C) formant un corps rigide avec la première portion (15B) de l'élément de protection (15) et présentant une deuxième extrémité (52) agissant sur la partie (45) de la pièce en forme de L (43) pour pousser la première portion (44) de cette dernière devant la cavité (17).

17. Aiguille en forme de canule selon la revendication 1, **caractérisée en ce que** la pièce de fermeture (43) de la première portion (15B) de l'élément de protection (15) est poussée de façon élastique pour porter la première (44) de ses portions (44, 45) dans une fente (132) pourvue dans la deuxième portion (15A) et ouvrant dans la cavité (17) de cette dernière.

18. Aiguille en forme de canule selon la revendication 15, **caractérisée en ce que** l'élément élastique (50) fait partie intégrante de la première portion (15B) de l'élément de protection.

19. Aiguille en forme de canule selon la revendication 15, **caractérisée en ce que** l'élément élastique est une partie constituante (15C) indépendamment accouplée et formant un corps rigide avec la première portion (15B) dudit élément de façon à agir sur la pièce en forme de L (43) de ce dernier.

20. Aiguille en forme de canule selon la revendication 18, **caractérisée en ce que** ledit élément élastique est au moins partiellement introduit dans le siège et/ou les logements (93, 98) de ladite première partie (15B).

21. Aiguille en forme de canule selon la revendication 1, **caractérisée en ce que** l'ouverture (19K) dans la partie (16A) du membre (16) faisant face au cathéter repose dans un plan coupant l'axe (W), cette ouverture (19K) étant inclinée vers l'axe (W) d'un angle équivalant à l'angle entre les parties (44, 45) de la pièce (43).

22. Aiguille en forme de canule selon la revendication 1, **caractérisée en ce que** les moyens anti-enroulement (55) sont prévus sur l'élément de protection (15) pour coopérer avec des contre-moyens (60) de la première portion (2) de l'aiguille en forme de canule pour éviter des mouvements de torsion relatifs entre cette dernière et ledit élément.

23. Aiguille en forme de canule selon la revendication 22, **caractérisée en ce que** les moyens anti-enroulement sont au moins un bras (55) sortant en saillie à partir de l'élément de protection et prévu de sorte qu'il coopère avec un renfoncement (60) prévu dans une bride d'extrémité (61) de ladite première portion (2) de l'aiguille en forme de canule (1).

24. Aiguille en forme de canule selon la revendication 23, **caractérisée en ce que** les moyens anti-enroulement comprenant le bras (55) présentent un accouplement à coup sec pour accoupler l'élément de protection (15) avec la portion frontale (2) de ladite aiguille en forme de canule (1).

25. Aiguille en forme de canule selon la revendication 22, **caractérisée en ce que** les moyens anti-enroulement sont des goupilles qui sont introduites dans des logements prévus dans la première et dans la deuxième portions (2, 3) de l'aiguille en forme de canule.
